# EUROPEAN PATENT SPECIFICATION

(11) **EP 4 395 874 B1**
(45) Date of publication and mention of the grant of the patent: **11.11.2026**
(21) Application number: 22865542.9
(22) Date of filing: 01.09.2022
(51) Int. Cl.: A61M 39/16, A61L 2/24, A61L 2/10, A61L 2/08, A61M 25/00

(54) **INTRAVENOUS CATHETER DISINFECTING DEVICE**
INTRAVENÖSE KATHETERDESINFEKTIONSVORRICHTUNG
DISPOSITIF DE DÉSINFECTION DE CATHÉTER INTRAVEINEUX

(30) Priority: 02.09.2021 US 202163240020 P
(43) Date of publication of application: 10.07.2024
(73) Proprietor: Becton, Dickinson and Company, Franklin Lakes, NJ 07417 (US)
(72) Inventor: RYAN, Kevin, M., Whitehouse Station, New Jersey 08889 (US); HUENERFAUTH, Angela, Marie, Morris Plains, New Jersey 07950 (US); PRASAD, Shishir, Ramsey, New Jersey 07446 (US)
(74) Representative: dompatent
(86) International application number: PCT/US2022/042291
(87) International publication number: WO 2023/034470

(56) References cited:
- WO-A1-02/102421
- US-A1- 2013 303 996
- US-A1- 2015 231 287
- US-A1- 2017 119 915
- US-A1- 2019 168 023
- US-A1- 2020 405 892
- US-B2- 9 295 742
- US-B2- 9 320 880

## Description

### BACKGROUND

Intravenous (IV) therapy may refer to a medical technique that is used to deliver fluids, medications, and/or nutrition directly into a body of a patient via an IV catheter inserted into a vein at an IV insertion site. IV therapy may be used for rehydration, to provide nutrition to a person who cannot consume food or water by mouth, and/or to administer medications or other medical therapy, such as blood products or electrolytes.

In some instances, components of an IV catheter for IV therapy may require a disinfection process. The disinfection process may involve the use of a topical disinfectant on the skin of the patient following a saline flush of the IV catheter at the IV injection site. For example, the disinfection process for an IV catheter may be to remove the IV catheter and components of the IV catheter and treating with a topical disinfectant, such as rubbing alcohol.

However, there may be significant drawbacks to removing the IV catheter and components of the IV catheter. For instance, removing the IV catheter and components of the IV catheter may increase exposure of these areas to potential infectious agents. In addition, it may also be important to minimize movement of the IV catheter and components of the IV catheter. In some instances, if the IV catheter is not properly secured in place, the IV catheter may be displaced from an intended location. Further, repeated movement of the IV catheter, to correctly position the IV catheter, can cause irritation of a blood vessel, disrupt proper introduction of medications to the patient, and/or increase the potential for bleeding or infection at the IV insertion site. If extensive movement occurs, the IV catheter may come out of the patient, interrupting delivery of medication and requiring re-insertion, potentially with a requirement for hospitalization of the patient. Accordingly, it is beneficial to use other methods of disinfection for catheter lines to remove risk of internal infection.

Therefore, there is an increasing need for disinfecting IV catheters, without the potential of introducing adverse effects to the patient.

US 2015/231287 A1 discloses systems, methods, and kits for sterilizing in vivo catheters using an optical fiber to deliver UV light. The method for sterilizing a catheter with at least a first lumen, includes inserting a distal end of a fiber optic cable into a fiber insertion port of a catheter connector attached to a hub of the first lumen, flushing the first lumen with fluid from a fluid source, inserting the fiber optic cable into the first lumen until a stopper of the fiber optic cable is adjacent to the fiber insertion port, providing light to the fiber optic cable from a light source after the fiber optic cable is inserted into the first lumen, withdrawing the fiber optic cable from the first lumen while the light is provided, and ceasing to provide light to the fiber optic cable after the fiber optic cable is withdrawn from the first lumen.

US 2013/303996 A1 discloses a system for disinfecting or sterilizing a catheter. A housing includes a chamber having lumens for transmitting one or more UV light sources through the lumens and into a Y-set or a transfer-set. The first UV light source and second UV light source emit UV light into the lumens in order to disinfect or sterilize the Y-set and the transfer-set.

### SUMMARY

Accordingly, provided are an improved IV catheter disinfecting device according to claim 1.

### BRIEF DESCRIPTION OF THE DRAWINGS

Additional advantages and details are explained in greater detail below with reference to the exemplary embodiments that are illustrated in the accompanying schematic figures, in which:
FIG. 1 is a diagram of a non-limiting embodiment of a catheter assembly and a disinfecting light source;
FIG. 2 is a diagram of the catheter assembly and the disinfecting light source shown in FIG. 1;
FIG. 3 is a diagram of a non-limiting embodiment of a catheter assembly with an extended view of a catheter;
FIG. 4 is an expanded view of the catheter assembly shown in FIG. 3;
FIG. 5 is a diagram of a non-limiting embodiment of a fiber optic connector;
FIG. 6 is a cross-sectional view of the fiber optic connector shown in FIG. 5;
FIG. 7 is a diagram of a non-limiting embodiment of a connector hub; and
FIG. 8 is a cross-sectional view of the connector hub of FIG. 7.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to embodiments or aspects as they are oriented in the drawing figures. However, it is to be understood that embodiments or aspects may assume various alternative variations and step sequences, except where expressly specified to the contrary. It is also to be understood that the specific devices and processes illustrated in the attached drawings, and described in the following specification, are simply non-limiting exemplary embodiments or aspects. Hence, specific dimensions and other physical characteristics related to the embodiments or aspects of the embodiments or aspects disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." As used in the specification and the claims, the singular form of "a," "an," and "the" include plural referents, such as unless the context clearly dictates otherwise. Additionally, Furthermore, as used herein, the terms "set" and "group" are intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some non-limiting embodiments or aspects may be described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

Provided are improved devices, systems, methods and products for disinfecting a catheter using a light source. Embodiments of the present disclosure may include a disinfection device that includes an optical fiber, a fiber optic connector, a light source and an intravenous catheter. The fiber optic connector may have a first end, a second end, and a channel, where the optical fiber may be positioned in the channel of the fiber optic connector. The light source may be coupled to the first end of the fiber optic connector and may be configured to provide light that disinfects an object, or a catheter. The intravenous catheter may have a plurality of channels, and may be coupled to the second end of the fiber optic connector. The optical fiber may further be positioned in a channel of the plurality of channels of the catheter. In some non-limiting embodiments, the light source may be from at least one light emitting diode (LED). In some non-limiting embodiments, the light from the light source may have a wavelength in the range of 400-450nm. In some non-limiting embodiments, a second channel of the plurality of channels of the catheter may be configured to allow fluid to flow, with the light source configured to provide light through the optical fiber to disinfect at least a portion of the second channel of the catheter. In some non-limiting embodiments, the optical fiber may be coupled to the light source and the second end of the optical fiber may be positioned in the first channel of the plurality of channels of the catheter. In some non-limiting embodiments, the second end of the optical fiber may have a light diffuser element. In some non-limiting embodiments, the disinfection device may have a sensor configured to determine an amount of time that light travels along the length of the optical fiber. In some non-limiting embodiments, the sensor may have a processor, the processor configured to receive a signal from the sensor, which provides data associated with the amount of time during which the light has traveled along the optical fiber. The processor then may determine whether the amount of time during which the light traveled along the optical fiber satisfies a threshold and may then determine a control operation based upon whether the amount of time during which the light traveled along the optical fiber satisfied the threshold. In some non-limiting embodiments, the threshold may be an amount of time based on a predetermined amount of time during which the light traveled along the entire length of the optical fiber. In some non-limiting embodiments, the disinfecting device may have a connecting hub to couple the second end of the fiber optic connector to the catheter. In some non-limiting embodiments, the disinfecting device may have an integrated safety interlock device, which may be coupled to the light source, and may be configured to prevent the light source from operating unless the light source is coupled to the first end of the fiber optic connector.

In this way, embodiments of the present disclosure allow for an IV catheter disinfecting device that disinfects IV catheters, without disturbing any devices used at the IV injection sites, such as an IV dressing that holds an IV line in place, and that lowers failure rates of IV catheters which may result from, for example, infections or blockages that form within a lumen of the user.

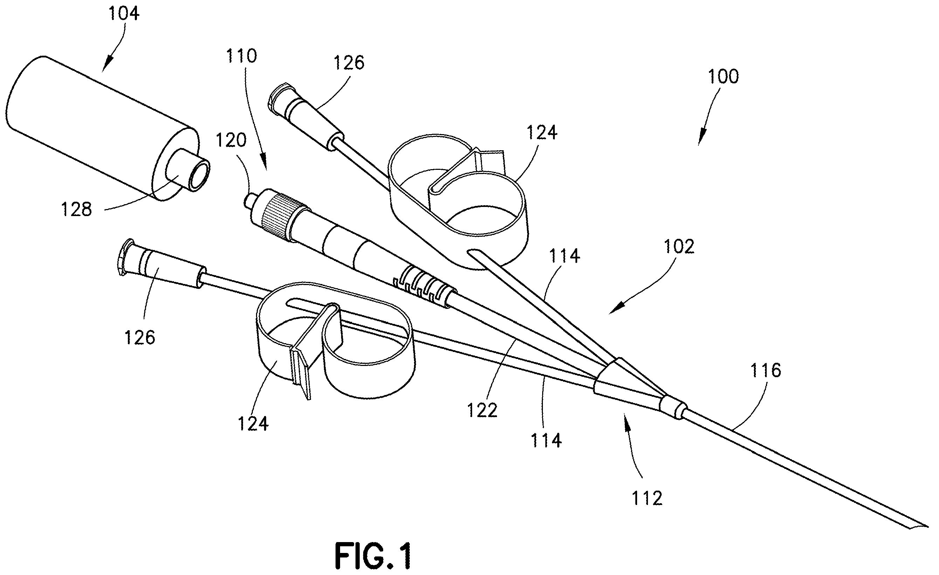

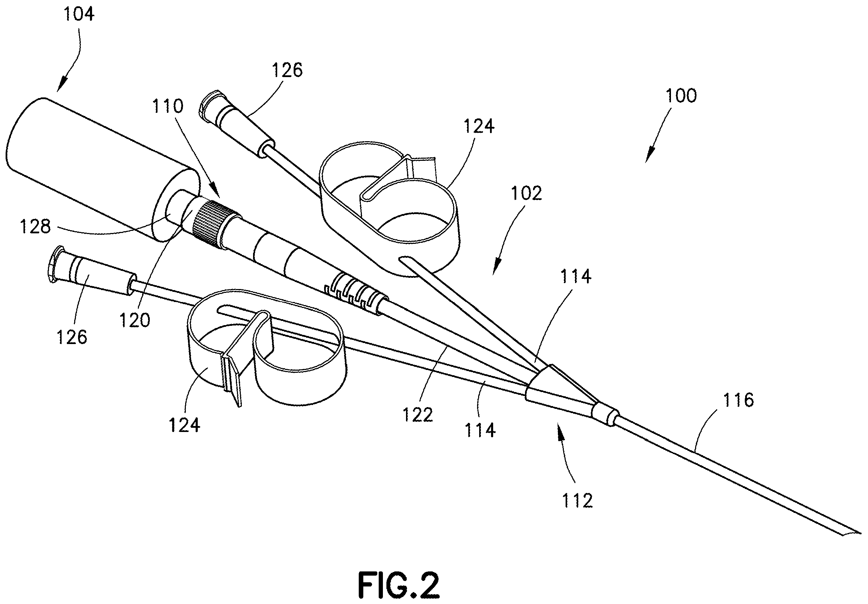

Referring now to FIGS. 1-2, FIGS. 1-2 are diagrams of an intravenous (IV) catheter disinfecting device 100. FIG. 1 is a diagram of IV catheter disinfecting device 100 with disinfecting light source 104 uncoupled from catheter assembly 102 and FIG. 2 is a diagram of IV catheter disinfecting device 100 with disinfecting light source 104 coupled to catheter assembly 102. As shown in FIGS. 1-2, IV catheter disinfecting device 100 may include catheter assembly 102 and disinfecting light source 104.

As further shown in FIGS. 1-2, catheter assembly 102 may include fiber optic connector 110, connector hub 112, extension lines 114, catheter 116, clamps 124, and coupling devices 126. As further shown in FIGS. 1-2, catheter 116 may be coupled to (e.g., connected to) connector hub 112 and extension lines 114 and fiber optic connector 110 may be coupled to connector hub 112. As further shown in FIGS. 1-2, fiber optic connector 110 may include first end 120 and second end 122.

In some non-limiting embodiments, disinfecting light source 104 may include one or more devices that are configured to provide light to disinfect a surface. For example, disinfecting light source 104 may include a device that is configured to provide light in a wavelength that is configured to disinfect a surface. In this way, disinfecting light source 104 may provide for disinfection (e.g., antimicrobial disinfection) of a surface (e.g., a surface of an IV catheter), based on the use of disinfecting light source 104. In some non-limiting embodiments, disinfecting light source 104 may include a light emitting diode (LED), a plurality of LEDs (e.g., an array of LEDs), a light bulb, a light-emitting electrochemical cell (LEC), a laser, and/or the like. In some non-limiting embodiments, disinfecting light source 104 may include one or more light emitting elements. For example, disinfecting light source 104 may include a plurality of emitting elements, where each light emitting element is an LED, a light bulb, an LEC, a laser, and/or the like.

In some non-limiting embodiments, extension line 114 may allow fluid to flow from a fluid source connected to extension 114 through extension line 114 and into a catheter 116. As further shown in FIGS. 1-2, extension line 114 may include coupling device 126 at an end of extension line 114. In some non-limiting embodiments, coupling device 126 may include one or more fittings, such as LuerLoc fluid fitting or Luer taper fluid fittings. In some non-limiting embodiments, coupling device 126 may be configured to connect to an external fluid source for administration of fluid through catheter assembly 102. As shown in FIGS. 1-2, each extension line 114 may include clamp 124. In some non-limiting embodiments, clamp 124 may be configured to restrict a flow of fluid through extension line 114. In some non-limiting embodiments, extension line 114 may be positioned in connector hub 112. For example, an end of extension line 114 (e.g., an end of extension line 114 opposite another end of extension line 114 to which coupling device 126 is attached) may be positioned in a fitting of connector hub 112.

In some non-limiting embodiments, IV catheter disinfecting device 100 may include one or more optical fibers. For example, IV catheter disinfecting device 100 may include one or more optical fibers, where disinfecting light source 104 is configured to provide light to the one or more optical fibers and the one or more optical fibers are configured to carry the light received from disinfecting light source 104 (e.g., carry the light received from disinfecting light source 104 to a location). In some non-limiting embodiments, an end of the one or more optical fibers may include a light diffuser element.

In some non-limiting embodiments, the one more optical fibers may be positioned in fiber optic connector 110 such that the one or more optical fibers may receive light provided by disinfecting light source 104 (e.g., when disinfecting light source is coupled to fiber optic connector 110) and transmit the light at certain positions along a length of the one or more optical fibers and/or at end of the one or more optical fibers. In some non-limiting embodiments, IV catheter disinfecting device 100 may have a first optical fiber and a second optical fiber. The first optical fiber may be configured to transmit light from disinfecting light source 104 to a fluid channel of catheter 116. In some non-limiting embodiments, the second optical fiber may be used for feedback to a sensor to detect a disconnection in the first optical fiber. In some non-limiting embodiments, the second optical fiber may also be used to provide feedback to a sensor or controller to adjust an intensity of light emitted from disinfecting light source 104.

In some non-limiting embodiments, disinfecting light source 104 may provide antimicrobial blue light (aBL), which may include electromagnetic radiation having a wavelength between 400-470 nm. Additionally or alternatively, disinfecting light source 104 may provide electromagnetic radiation having a wavelength between 630-680 nm, which may be referred to as red light. Additionally or alternatively, disinfecting light source 104 may provide electromagnetic radiation having a wavelength between 10-400 nm, which may include electromagnetic radiation in the ultraviolet (UV) spectrum. For example, disinfecting light source 104 may provide electromagnetic radiation having a wavelength between 100-280 nm (e.g., the UVC spectrum), electromagnetic radiation having a wavelength between 280-315 nm (e.g., the UVB spectrum), and/or electromagnetic radiation having a wavelength between 315-400 nm (e.g., the UVA spectrum).

In some non-limiting embodiments, disinfecting light source 104 may have a sensor (not shown) which may include one or more devices that are configured to detect a disconnection (e.g., a cut, a break, etc.) in the optical fiber or a disconnection of disinfecting light source 104 from fiber optic connector 110. For example, the sensor may be incorporated into disinfecting light source 104 and include one or more devices that are configured to receive feedback from an optical fiber to determine a discontinuation in the light. In this way, in the case that a disconnection is detected, disinfecting light source 104 may not produce light. In some non-limiting embodiments, the sensor may detect a disconnection based on time, color, and/or conductivity (e.g., a difference in conductivity). In some non-limiting embodiments, sensor may include one or more photoelectric sensors, one or more laser sensors, one or more ultrasonic sensors, one or more color sensors, one or more contact sensors, one or more proximity sensors, one or more image sensors, and/or the like. In some non-limiting embodiments, IV catheter disinfecting device 100 may include one or more sensors.

In some non-limiting embodiments, disinfecting light source 104 may be controlled based on a sensor. For example, a controller (not shown) may receive a signal from the sensor and the controller may cause disinfecting light source 104 to activate or deactivate based on the signal received from the sensor.

In some non-limiting embodiments, IV catheter disinfecting device 100 may include safety device associated with an optical fiber, such as a sensor that is used to determine if there is a discontinuation in an optical fiber. In one example, the sensor may be configured to determine an amount of time during which light traveled along an optical fiber. In such an example, the sensor may transmit a signal includes data associated with the amount of time during which the light traveled along the optical fiber and the controller may receive the signal from the sensor. Accordingly, the controller may determine whether the amount of time during which the light traveled along the optical fiber satisfies a threshold. In some non-limiting embodiments, the threshold may include a threshold value of an amount of time based on a predetermined amount of time for light to travel along the length of the optical fiber. Further, the controller may determine a control operation based on determining whether the amount of time during which the light traveled along the optical fiber satisfies the threshold. In some non-limiting embodiments, the controller may determine a control operation for disinfecting light source 104 based on determining that the amount of time during which the light traveled along the optical fiber satisfies the threshold. For example, the controller may activate (e.g., continue to activate, forego deactivating, etc.) disinfecting light source 104 based on determining that the amount of time during which the light traveled along the optical fiber satisfies the threshold. In some non-limiting embodiments, the controller may determine a control operation for disinfecting light source 104 based on determining that the amount of time during which the light traveled along the optical fiber does not satisfy the threshold. For example, the controller may deactivate (e.g., prevent from activating, forego activating, etc.) disinfecting light source 104 based on determining that the amount of time during which the light traveled along the optical fiber does not satisfy the threshold.

As referred to herein, a controller may include a device configured to control disinfecting light source 104. For example, the controller may include a device configured to control (e.g., control the activation and/or deactivation of) disinfecting light source 104 based on information received from one or more sensors. In some non-limiting embodiments, the controller may include a processor, such as a central processing unit (CPU), a microcontroller, an integrated circuit (IC), and/or the like. In some non-limiting embodiments, the controller may include a switch device, such as an electrical switch.

In some non-limiting embodiments, disinfecting light source 104 may be controlled based on a time interval (e.g., a predetermined time interval, a time interval associated with a disinfection process, etc.). In one example, a controller may cause disinfecting light source 104 to activate based on an initiation of the time interval (e.g., based on the activation of a time counter for the time interval). Additionally or alternatively, the controller may cause one or more light elements of disinfecting light source to deactivate based on an expiration of the time interval. In some non-limiting embodiments, disinfecting light source 104 may be configured to be deactivated based on an expiration of the time interval. In some non-limiting embodiments, the time interval may be selected by a user of IV catheter disinfecting device 100 (e.g., a patient, a doctor, etc.). For example, the time interval may be selected by the user via a user input device, such as a physical component (e.g., a dial, a switch, a button, etc.) or a user interface (e.g., a graphical user interface, an element of a user interface, etc.). In some non-limiting embodiments, the time interval may be a predetermined time interval (e.g., a preprogrammed time interval) that is stored in a memory of IV catheter disinfecting device 100 and that is retrieved by a controller prior to activation of disinfecting light source 104.

In some non-limiting embodiments, disinfecting light source 104 may be controlled based on a time interval and a sensor. For example, a controller may prevent the initiation of the time interval (e.g., the time interval during which disinfecting light source 104 is activated) unless a signal (e.g., an activation signal) is received from the sensor. In some non-limiting embodiments, the controller may initiate the time interval based on receiving the signal from the sensor. In another example, the controller may prevent the initiation of the time interval unless a signal is received from the sensor. In some non-limiting embodiments, the controller may initiate the time interval based on receiving a signal from the sensor that indicates that disinfecting light source 104 is coupled to fiber optic connector 110.

In some non-limiting embodiments, disinfecting light source 104 may be controlled based on a predetermined sequence. For example, one or more light elements of disinfecting light source 104 may be activated and/or deactivated based on the predetermined sequence. In some non-limiting embodiments, disinfecting light source 104 may be controlled based on an input from a user. For example, one or more light elements of disinfecting light source 104 may be activated based on the input from the user. In some non-limiting embodiments, the input from the user may overwrite a predetermined sequence during which disinfecting light source 104 may be activated. For example, one or more light elements of disinfecting light source 104 may be activated based on the predetermined sequence and during the predetermined sequence, an input from the user is received.

As shown in FIG. 2, disinfecting light source 104 may be coupled to catheter assembly 102. For example, first end 120 of fiber optic connector 110 may be coupled to end 128 of disinfecting light source 104. In this way, disinfecting light source 104 may be selectively coupled to or uncoupled from catheter assembly 102 by a user as necessary. In some non-limiting embodiments, fiber optic connector 110 may be coupled to disinfecting light source 104 via a fastening mechanism. For example, fiber optic connector 110 may be coupled to disinfecting light source 104 via a threaded connection (e.g., a threaded connection between end 128 of disinfecting light source 104 and first end 120 of fiber optic connector 110). In another example, fiber optic connector 110 may be coupled to disinfecting light source 104 via a friction fit connection (e.g., a friction fit connection between end 128 of disinfecting light source 104 and first end 120 of fiber optic connector 110). In another example, fiber optic connector 110 may be coupled to disinfecting light source 104 via a locking connection, such as a latching connection, an interlocking connection, and/or the like (e.g., a locking connection between end 128 of disinfecting light source 104 and first end 120 of fiber optic connector 110). In some non-limiting embodiments, one or more light elements of disinfecting light source 104 may be coupled to first end 120 of fiber optic connector 110.

In some non-limiting embodiments, IV catheter disinfecting device 100 may include an integrated safety interlock device. For example, disinfecting device 100 may include an integrated safety interlock device that is configured to prevent disinfecting light source 104 from operating unless disinfecting light source 104 is coupled (e.g., properly coupled) to fiber optic connector 110. In some non-limiting embodiments, the integrated safety interlock device may be configured to prevent disinfecting light source 104 from operating unless disinfecting light source 104 is coupled to fiber optic connector 110 such that all light provided by disinfecting light source 104 is transmitted to an optical fiber without allowing any light to escape to a surrounding environment of disinfecting light source 104. In this way, the integrated safety interlock device may prevent the light provided by disinfecting light source 104 from shown on unintended locations, including sensitive areas of users of IV catheter disinfecting device 100. In some non-limiting embodiments, the integrated safety interlock device may be a component of a connection between fiber optic connector 110 and disinfecting light source 104. In some non-limiting embodiments, the integrated safety interlock device may include a contact sensor, a contact switch (e.g., a sensor connected to a switch that is open state or a closed state based on whether contact is detected), a conductivity sensor, and/or the like.

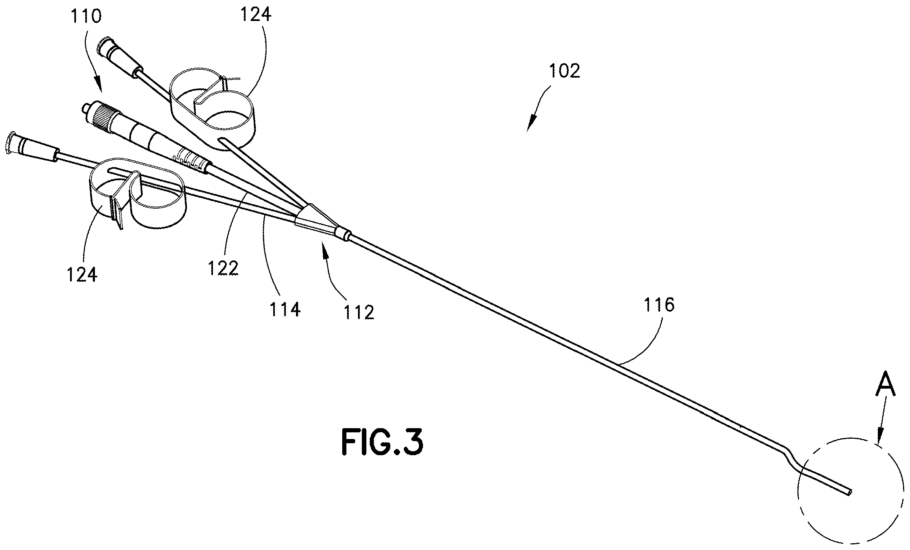

Referring now to FIG. 3, FIG. 3 shows a diagram of catheter assembly 102. In some non-limiting embodiments, catheter 116 of catheter assembly 102 may have a predetermined length to allow for catheter 116 to be inserted into a lumen of a blood vessel of a user to allow for intravenous delivery of fluids.

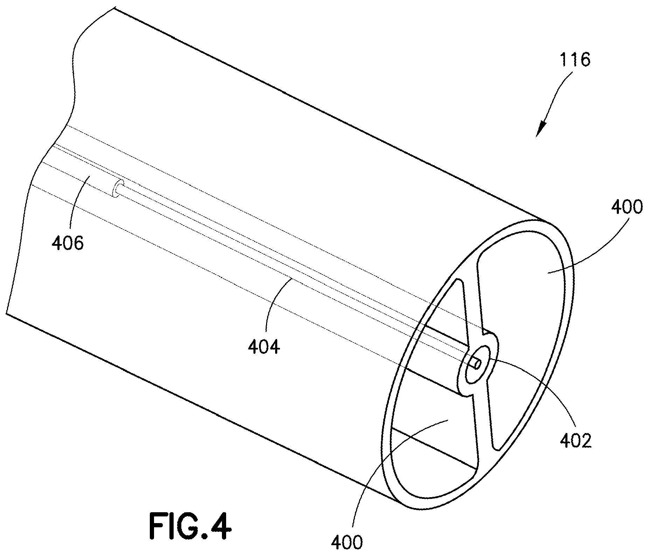

As shown in FIG. 4, FIG. 4 is an enlarged view of in catheter 116. As shown in FIG. 4, catheter 116 may include fluid channels 400 (e.g., channels in which fluid flows) and optical fiber channel 402. In some non-limiting embodiments, optical fiber 404 may be positioned in optical fiber channel 402. In some non-limiting embodiments, optical fiber 404 may include sheath 406. In some non-limiting embodiments, sheath 406 may be provided on only a portion of a length of optical fiber 404. In some non-limiting embodiments, sheath 406 may include a material that prevents a transmission of light from optical fiber 404 to an environment around optical fiber 404. In this way, sheath 406 may allow light provided by optical fiber 404 to be focused in a specific area (e.g., desired area) without providing the light to other areas. In some non-limiting embodiments, each fluid channel 400 may receive fluid that has travel through extension line 114 (e.g., extension one 114 which may be coupled to catheter 116 via connector hub 112). In some non-limiting embodiments, optical fiber channel 402 may contain optical fiber 404 to allow for optical fiber 404 to provide light from disinfecting light source 104 to an interior of each fluid channel 400. In some non-limiting embodiments, an end of optical fiber 404 (e.g., an end of optical fiber 404 positioned in optical fiber channel 402 of catheter 116 may include a light diffuser element).

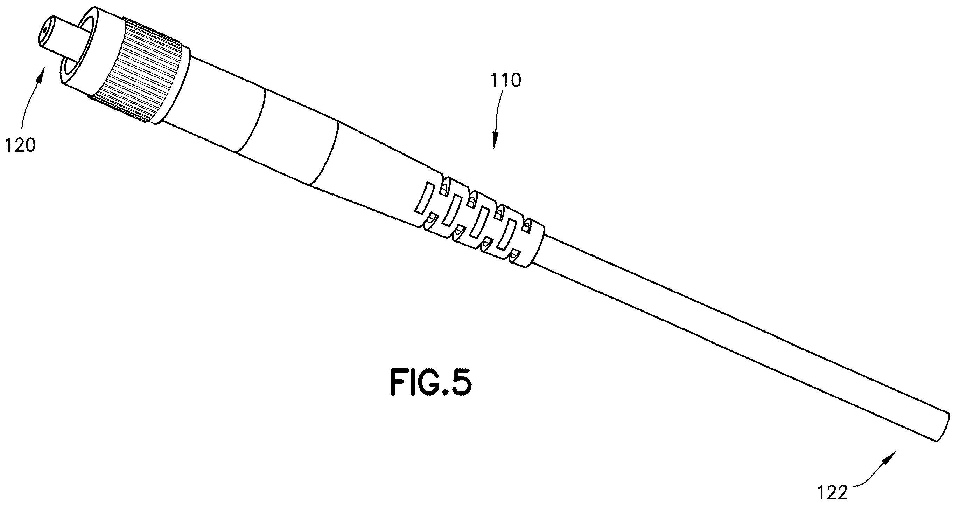

Referring now to FIG. 5, FIG. 5 is a diagram of a non-limiting embodiment of fiber optic connector 110. In some non-limiting embodiments, fiber optic connector 110 may have first end 120 and second end 122. In some non-limiting embodiments, first end 120 and second end 122 may be configured to be coupled to other objects such as catheter assembly 102 and/or disinfecting light source 104. As shown in FIG. 5, fiber optic connector 110 may be tapered in shape, with the body wider closest to the first end 120 and tapering to a narrow width towards the second end 122. In this way, fiber optic connector 110 may be coupled to connector hub 112 while allowing for ergonomic comfort of assembly for the user.

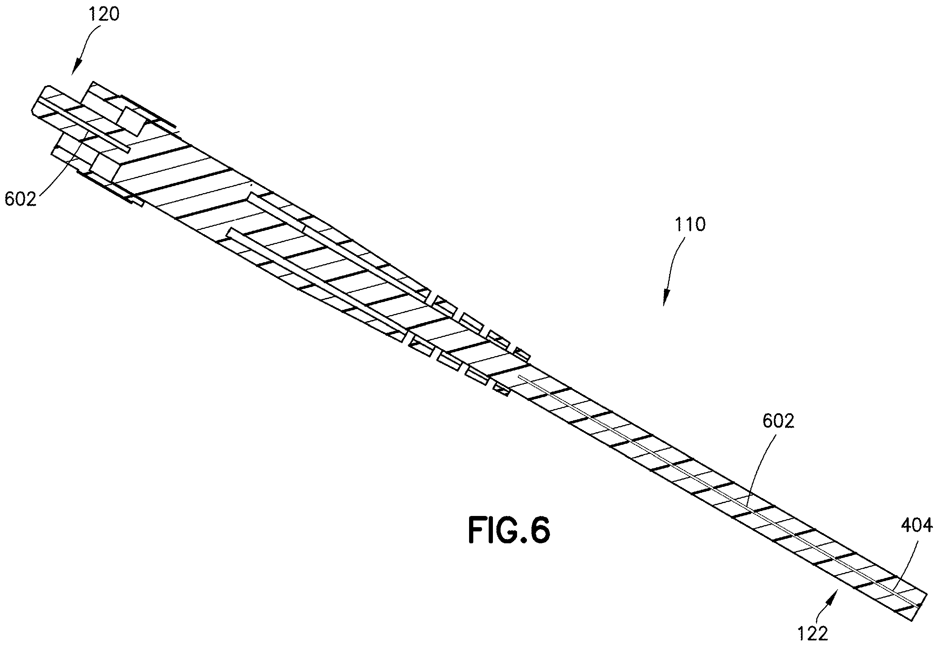

Referring now to FIG. 6, FIG. 6 is a cross-sectional view of fiber optic connector 110. In some non-limiting embodiments, fiber optic connector 110 may include channel 602 which may extend the length of fiber optic connector 110 from first end 120 to second end 122. In some non-limiting embodiments, optical fiber 404 is positioned in channel 602. In some non-limiting embodiments, optical fiber 404 may extend beyond the total length of fiber optic connector 110. In some further non-limiting embodiments, optical fiber 404 may extend beyond a length of fiber optic connector 110 (e.g., and be positioned in optical fiber channel 402 of catheter 116). In some non-limiting embodiments, fiber optic connector 110 may include a plurality of optical fibers 404 positioned in channel 602.

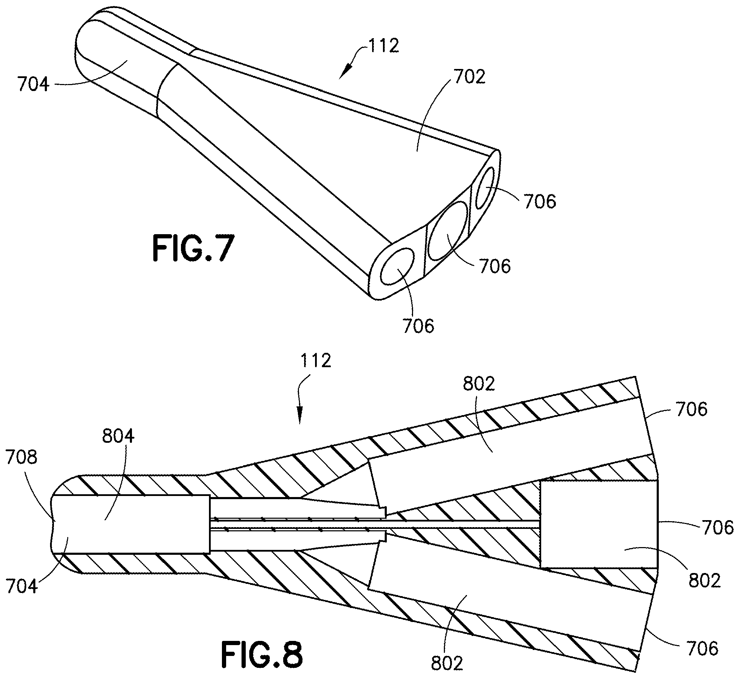

Referring now to FIG. 7, FIG. 7 is a diagram of connector hub 112. In some non-limiting embodiments, connector hub 112 may have a receiving end 702 and an output end 704. For example, receiving end 702 may be configured to position components of catheter assembly 102 in connector hub 112. In some non-limiting embodiments, receiving end 702 may have a plurality of inlets 706 configured to receive components of catheter assembly 102 (e.g., extension lines 114 and/or fiber optic connector 110). In some non-limiting embodiments, connector hub 112 may have an output end 704.

Referring now to FIG. 8, FIG. 8 is a cross-sectional view of connector hub 112. As shown in FIG. 8, connector hub 112 may include plurality of inlets 706 that correspond to a plurality of channels 802 that come together and are connected to channel 804. As further shown in FIG. 8, output end 704 may include outlet 708, which provides a single outlet for connector hub 112. In some non-limiting embodiments, catheter 116 may be positioned in outlet 708. In some non-limiting embodiments, channels 802 continue from receiving end 702 to channel 804 at output end 704. In some non-limiting embodiments, connector hub 112 may include a plurality of channels (e.g., 2 channels, 3 channels, 4 channels, etc.) which taper to a single channel in the output end 704.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within scope of the appended claims. For example, it is to be understood that the present disclosure contemplates that, to the extent possible, one or more features of any embodiment or aspect can be combined with one or more features of any other embodiment or aspect. In fact, many of these features can be combined in ways not specifically recited in the claims and/or disclosed in the specification. Although each dependent claim listed below may directly depend on only one claim, the disclosure of possible implementations includes each dependent claim in combination with every other claim in the claim set.

## Claims

1. A device comprising:
an optical fiber;
a fiber optic connector having a first end, a second end, and a channel, wherein the optical fiber is positioned in the channel of the fiber optic connector;
a light source coupled to the first end of the fiber optic connector, wherein the light source is configured to provide light to the optical fiber, and wherein the light source is configured to provide light that disinfects an object;
an intravenous (IV) catheter having a plurality of channels, wherein the catheter is coupled to the second end of the fiber optic connector, and wherein the optical fiber is positioned in a first channel of the plurality of channels of the catheter; and
an integrated safety interlock device, wherein the integrated safety interlock device is coupled to the light source, wherein the integrated safety interlock device is a sensor configured to determine if there is a disconnection in the optical fiber, and wherein the integrated safety interlock device is configured to prevent the light source from operating unless the light source is coupled to the first end of the fiber optic connector such that all light provided by the light source is transmitted to the optical fiber without allowing light to escape to a surrounding environment of the light source.

2. The device of claim 1, wherein a second channel of the plurality of channels of the catheter is configured to provide a channel in which a fluid flows, and wherein the optical fiber is positioned in the first channel to provide light from the light source that disinfects at least a portion of the second channel of the catheter.

3. The device of claim 1, wherein the optical fiber has a first end and a second end, wherein the first end of the optical fiber is coupled to the light source and wherein the second end of the optical fiber is positioned with the first channel of the plurality of channels of the catheter.

4. The device of claim 3, wherein the second end of the optical fiber comprises a light diffuser element.

5. The device of claim 1, further comprising:
a sensor configured to determine an amount of time during which the light traveled along the optical fiber.

6. The device of claim 5, further comprising:
a processor, wherein the processor is configured to:
receive a signal from the sensor, wherein the signal comprises data associated with the amount of time during which the light traveled along the optical fiber;
determine whether the amount of time during which the light traveled along the optical fiber satisfies a threshold; and
determine a control operation based on determining whether the amount of time during which the light traveled along the optical fiber satisfies the threshold.

7. The device of claim 6, wherein the threshold comprises a threshold value of an amount of time based on a predetermined amount of time during which the light traveled along an entire length of the optical fiber.

8. The device of claim 1, wherein the light source comprises at least one light emitting diode (LED).

9. The device of claim 1, wherein the light source provides light having a wavelength in a range of 400-450 nm.

10. The device of claim 1, further comprising a connector hub, and wherein the catheter is coupled to the second end of the fiber optic connector via the connector hub.

## Patentansprüche

1. Vorrichtung, die aufweist:
einen Lichtwellenleiter;
einen Lichtwellenleiterstecker mit einem ersten Ende, einem zweiten Ende und einem Kanal, wobei der Lichtwellenleiter in dem Kanal des Lichtwellenleitersteckers positioniert ist;
eine Lichtquelle, die mit dem ersten Ende des Lichtwellenleitersteckers gekoppelt ist, wobei die Lichtquelle so ausgebildet ist, dass sie Licht an den Lichtwellenleiter bereitstellt, und wobei die Lichtquelle so ausgebildet ist, dass sie Licht bereitstellt, das ein Objekt desinfiziert;
einen intravenösen (IV) Katheter mit einer Vielzahl von Kanälen, wobei der Katheter mit dem zweiten Ende des Lichtwellenleitersteckers gekoppelt ist, und wobei der Lichtwellenleiter in einem ersten Kanal der Vielzahl von Kanälen des Katheters positioniert ist; und
eine integrierte Sicherheitsverriegelungsvorrichtung, wobei die integrierte Sicherheitsverriegelungsvorrichtung mit der Lichtquelle gekoppelt ist, wobei die integrierte Sicherheitsverriegelungsvorrichtung ein Sensor ist, der so ausgebildet ist, dass er feststellt, ob eine Unterbrechung im Lichtwellenleiter vorliegt, und wobei die integrierte Sicherheitsverriegelung so ausgebildet ist, dass sie den Betrieb der Lichtquelle verhindert, sofern die Lichtquelle nicht mit dem ersten Ende des Lichtwellenleitersteckers verbunden ist, sodass das gesamte von der Lichtquelle bereitgestellte Licht an den Lichtwellenleiter übertragen wird, ohne dass Licht in eine Umgebung der Lichtquelle austreten kann.

2. Vorrichtung nach Anspruch 1, wobei ein zweiter Kanal der Vielzahl von Kanälen des Katheters so ausgebildet ist, dass er einen Kanal bereitstellt, in dem ein Fluid fließt, und wobei der Lichtwellenleiter in dem ersten Kanal positioniert ist, um Licht von der Lichtquelle bereitzustellen, das zumindest einen Teil des zweiten Kanals des Katheters desinfiziert.

3. Vorrichtung nach Anspruch 1, wobei der Lichtwellenleiter ein erstes Ende und ein zweites Ende aufweist, wobei das erste Ende des Lichtwellenleiters mit der Lichtquelle gekoppelt ist, und wobei das zweite Ende des Lichtwellenleiters mit dem ersten Kanal der Vielzahl von Kanälen des Katheters positioniert ist.

4. Vorrichtung nach Anspruch 3, wobei das zweite Ende des Lichtwellenleiters ein Lichtdiffusorelement aufweist.

5. Vorrichtung nach Anspruch 1, die ferner aufweist:
ein Sensor, der so ausgebildet ist, dass er die Zeitspanne ermittelt, in der sich das Licht entlang des Lichtwellenleiters fortbewegt hat.

6. Vorrichtung nach Anspruch 5, die ferner aufweist:
einen Prozessor, wobei der Prozessor für Folgendes ausgebildet ist:
Empfangen eines Signals vom Sensor, wobei das Signal Daten enthält, die sich auf die Zeitspanne beziehen, in der sich das Licht entlang des Lichtwellenleiters fortbewegt hat;
Feststellen, ob die Zeitspanne, in der sich das Licht entlang des Lichtwellenleiters fortbewegt hat, eine Schwelle erfüllt; und
Bestimmen eines Steuervorgangs auf der Grundlage der Feststellung, ob die Zeitspanne, in der sich das Licht entlang des Lichtwellenleiters fortbewegt hat, die Schwelle erfüllt.

7. Vorrichtung nach Anspruch 6, wobei die Schwelle einen Schwellenwert einer Zeitspanne umfasst, der auf einer vorbestimmten Zeitspanne basiert, in der das Licht eine gesamte Länge des Lichtwellenleiters zurückgelegt hat.

8. Vorrichtung nach Anspruch 1, wobei die Lichtquelle mindestens eine Leuchtdiode (LED) aufweist.

9. Vorrichtung nach Anspruch 1, wobei die Lichtquelle Licht mit einer Wellenlänge im Bereich von 400-450 nm liefert.

10. Vorrichtung nach Anspruch 1, die ferner einen Steckeransatz aufweist, wobei der Katheter über den Steckeransatz mit dem zweiten Ende des Lichtwellenleitersteckers verbunden ist.

## Revendications

1. Dispositif comprenant :
une fibre optique ;
un connecteur de fibre optique ayant une première extrémité, une deuxième extrémité et un canal, dans lequel la fibre optique est positionnée dans le canal du connecteur de fibre optique ;
une source de lumière couplée à la première extrémité du connecteur de fibre optique, dans lequel la source de lumière est configurée pour fournir de la lumière à la fibre optique, et dans lequel la source de lumière est configurée pour fournir de la lumière qui désinfecte un objet ;
un cathéter intraveineux (IV) ayant une pluralité de canaux, dans lequel le cathéter est couplé à la deuxième extrémité du connecteur de fibre optique, et dans lequel la fibre optique est positionnée dans un premier canal de la pluralité de canaux du cathéter ; et
un dispositif de verrouillage de sécurité intégré, dans lequel le dispositif de verrouillage de sécurité intégré est couplé à la source de lumière, dans lequel le dispositif de verrouillage de sécurité intégré est un capteur configuré pour déterminer s'il y a une déconnexion dans la fibre optique, et dans lequel le dispositif de verrouillage de sécurité intégré est configuré pour empêcher la source de lumière de fonctionner sauf si la source de lumière est couplée à la première extrémité du connecteur de fibre optique de sorte que toute la lumière fournie par la source de lumière soit transmise à la fibre optique sans permettre à la lumière de s'échapper vers un milieu environnant de la source de lumière.

2. Dispositif selon la revendication 1, dans lequel un deuxième canal de la pluralité de canaux du cathéter est configuré pour fournir un canal dans lequel un fluide s'écoule, et dans lequel la fibre optique est positionnée dans le premier canal pour fournir de la lumière provenant de la source de lumière qui désinfecte au moins une partie du deuxième canal du cathéter.

3. Dispositif selon la revendication 1, dans lequel la fibre optique comporte une première extrémité et une deuxième extrémité, dans lequel la première extrémité de la fibre optique est couplée à la source de lumière et dans lequel la deuxième extrémité de la fibre optique est positionnée avec le premier canal de la pluralité de canaux du cathéter.

4. Dispositif selon la revendication 3, dans lequel la deuxième extrémité de la fibre optique comprend un élément diffuseur de lumière.

5. Dispositif selon la revendication 1, comprenant en outre :
un capteur configuré pour déterminer une durée pendant lequel la lumière s'est déplacée le long de la fibre optique.

6. Dispositif selon la revendication 5, comprenant en outre :
un processeur, dans lequel le processeur est configuré pour :
recevoir un signal provenant du capteur, dans lequel le signal comprend des données associées à la durée pendant lequel la lumière s'est déplacée le long de la fibre optique ;
déterminer si la durée pendant lequel la lumière s'est déplacée le long de la fibre optique satisfait un seuil ; et
déterminer une opération de commande sur la base de la détermination du fait que la durée pendant lequel la lumière s'est déplacée le long de la fibre optique satisfait le seuil.

7. Dispositif selon la revendication 6, dans lequel le seuil comprend une valeur seuil d'une durée basée sur une durée prédéterminé pendant lequel la lumière s'est déplacée le long de toute la longueur de la fibre optique.

8. Dispositif selon la revendication 1, dans lequel la source de lumière comprend au moins une diode électroluminescente (DEL).

9. Dispositif selon la revendication 1, dans lequel la source de lumière fournit de la lumière ayant une longueur d'onde dans une plage de 400 à 450 nm.

10. Dispositif selon la revendication 1, comprenant en outre un moyeu de connecteur, et dans lequel le cathéter est couplé à la deuxième extrémité du connecteur de fibre optique via le moyeu de connecteur.
